# NEW EUROPEAN PATENT SPECIFICATION

(11) **EP 1 765 420 B2**
(45) Date of publication and mention of the opposition decision: **28.06.2017**
(45) Mention of the grant of the patent: 22.02.2012
(21) Application number: 05758807.1
(22) Date of filing: 20.06.2005
(51) Int. Cl.: A61L 2/16, A61L 2/18, C11D 1/825, A01N 47/44, A61K 31/14, A61K 31/155, C07C 279/26

(54) **SANITIZING COMPOSITION**
SANITISIERUNGSZUSAMMENSETZUNG
COMPOSITION DESINFECTANTE

(30) Priority: 25.06.2004 GB 0414244
(43) Date of publication of application: 28.03.2007
(73) Proprietor: Ebiox Limited, Warrington WA3 7QZ (GB)
(72) Inventor: MACGREGOR, Keith, Martin 40 Main Street, Leeds LS1 4HR (GB)
(74) Representative: chapman + co
(86) International application number: PCT/GB2005/002430
(87) International publication number: WO 2006/000756

(56) References cited:
- WO-A-2004/101432
- US-A- 6 083 457
- US-A1- 2003 080 317
- US-A1- 2004 120 916
- US-B1- 6 663 902
- DATABASE WPI Section Ch, Week 199021 Derwent Publications Ltd., London, GB; Class C01, AN 1990-161317 XP002341529 & JP 02 104509 A (DAICEL CHEM IND LTD) 17 April 1990 (1990-04-17)

## Description

The present invention relates to the use of a sanitizing composition, particularly to a sanitizing composition used in medical facilities.

A known problem in medical facilities is the spread of pathogens. These pathogens - typically bacteria - may stem from only one patient, or region, in such a facility but if not quickly eradicated may spread to contaminate other patients, or regions. One known phenomenon which assists pathogens in spreading is the formation of a biofilm.

Biofilms form when micro-organisms adhere to a surface. They grow and become a culture medium for more micro-organisms. A biofilm can be formed by a single species or micro-organism, for example a bacterium, fungus, algae, or protozoa. However, biofilms may often be formed by multiple species of micro-organism; for example they may often be formed of multiple species of bacteria. Alternatively or additionally they may be formed from debris. The debris may be from living organisms, for example sebum or dead skin cells. Alternatively or additionally the debris may be from inanimate sources, for example corrosion products.

The formation of biofilms is a serious problem with grave consequences in medical facilities where, inevitably, harmful pathogens such as Methicillin Resistant *Staphylococcus aureus* (MRSA), *Legionella* (responsible for legionnaires disease) and *Escherichia coli* (*E. coli*) may be present. The need to develop technologies to stop these bacteria from spreading is paramount.

Accordingly, there is a continued need for a means of combating pathogens in medical facilities. In particular there is a need to destroy and prevent the formation of biofilms in which the pathogens may flourish.

US-A-2004/0120916 describes a contact lens care solution comprising taurine, a liquid aqueous medium, an antimicrobial component, a surfactant and a buffer to keep the pH within the desired physiological range of about 6 to about 9. The solution prevents losses in ocular tissue membrane integrity during contact lens wear.

US 6,663,902B describes a biocidal composition designed for the generation of chlorine dioxide,

WO-A-01/23411 describes an antimicrobial, hard surface cleaning composition comprising (a) cationic antimicrobial additive; (b) nitrogen-containing polymer; and (c) surfactant.

According to the present invention there is provided the use of a sanitizing composition both to kill or disable pathogens present at a locus and to destroy or degrade biofilm present at a locus, the composition comprising, in aqueous solution:
at least one surfactant, present in an amount in the range 0.05-5% w/w;
antimicrobial agents;
and at least one acid;
wherein:
   said at least one surfactant comprises at least one non-ionic surfactant;
   the antimicrobial agents comprise a quaternary ammonium compound present in an amount in the range 0.001-3% w/w, and a biguanide compound present in an amount in the range 0.001-2% w/w;
   the pH of the composition is in the range 2-5; and
   the at least one acid comprises citric acid.

Examples of suitable non-ionic surfactants include but are not restricted to; alkoxylated alcohols including ethoxylated and propoxylated fatty alcohols as well as ethoxylated and propoxylated alkyl phenols, preferably having alkyl groups with carbon chain length of from 7 to 16, more preferably a to 13.

A preferred group of non-ionic surfactants is alkylphenol ethoxylates (APEs). The structure of suitable alkylphenol ethoxylates usually comprises an alkylphenol with a side chain of several ethoxylate groups. The alkyl group is typically a branched nonyl-, octyl- or dodecyl-chain. Examples of suitable alkylphenol ethoxylates include, but are not restricted to: poly (oxy-1,2-ethanol) alpha-(nonylphenol)-omega-hydroxy; Ultranex NP95; Surfonic N95; Polytergent B300; Tergitol NP9; Synperonic NP9.5; Marlophen 89.5. A particularly preferred non-ionic surfactant is a nonylphenol ethoxylate, especially that sold under the trade mark Synperonic N.

As noted above the antimicrobial agents comprise a quaternary ammonium compound. Preferably, such an antimicrobial agent has the following general formula: 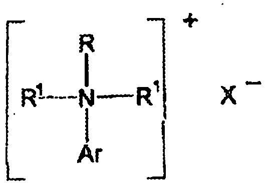 where Ar is an optionally substituted aryl or heteroaryl group, R is any C6 or above unsubstituted branched or linear alkyl group, each group R1 is independently selected from any C1 to C4 branched or unbranched unsubstituted alkyl, and X is a halide anion.

Optional substituents of an acryl or heteroaryl group Ar include halo, cyano, and C1-C4 alkoxy and C1-C4 haloalkyl groups. There may suitably be 1-3 substituents. Preferably, however, Ar is an unsubstituted aryl or heteroaryl group.

Preferably, Ar is selected from optionally substituted phenyl, benzyl, napthyl and pyridyl groups. Most preferably, Ar is an optionally substituted aryl group. Most preferably Ar is an optionally substituted benzyl group.

Preferably, R is any C8 or above unsubstituted branched or linear alkyl group. More preferably, R is any C12 to C20 unsubstituted branched or linear alkyl group. Most preferably, R is any C12 to C20 unsubstituted linear alkyl group. In a particularly preferred embodiment, R is an unbranched unsubstituted C18 alkyl group.

Preferably, R1 are each independently selected from methyl, ethyl, propyl, butyl and isopropyl. More preferably, R1 are each methyl groups.

Preferably, X is a chloride, bromide or iodide anion. Most preferably, X is a chloride anion.

Preferably, the antimicrobial compound comprises benzalkonium chloride (BAC), or may be a derivative thereof.

A quaternary ammonium compound is preferably present in an amount in an amount in the range 0.01 - 1 % w/w, more preferably in an amount in the range 0.05 - 0.5 % w/w, and most preferably in an amount in the range 0.1 - 0.4 % w/w; and especially, at a concentration of about 0.2 - 0.3 % w/w.

As noted above, the antimicrobial agents comprise a biguanide compound.

By "biguanide compound" we mean a chemical having the following functional group: 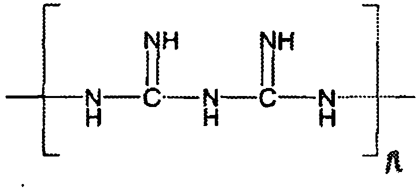 where n = 1 or 2.

When n = 2, the alpha nitrogen becomes tetravalent and hence positively charged. In this case, the charge may be equalised by any anion, preferably a halide anion such as chloride, bromide or iodide.

When n = 2, the alpha nitrogen becomes tetravalent and hence positively charged. In this case, the charge may be equalised by any anion, preferably a halide anion such as chloride, bromide or iodide.

Preferably, a biguanide antimicrobial agent is a polymeric biguanide compound. A particularly preferred polymeric biguanide compound is polyhexamethylenebiguanide (PHMB), or derivatives thereof.

A biguanide antimicrobial agent is present in an amount in the range 0.001-2% W/N and preferably present in an amount in the range 0.005 - 1 % w/w, more preferably in an amount in the range 0.01 - 0.5 % w/w, and most preferably in an amount in the range 0.02 - 0.2 % w/w; and especially, in an amount in the range 0.05 - 0.15 % w/w.

Preferably the quaternary ammonium antimicrobial agent and the biguanide antimicrobial agent are present in amounts to satisfy three numerical definitions: at least one of the numerical definition which applies to the quaternary ammonium antimicrobial agent itself; at least one of the numerical definition which applies to the biguanide antimicrobial agent itself; and at least one of the numerical definitions which applies to the "at least one antimicrobial agent"

Preferably the at least one acid is present in an amount in the range 0.01 - 5 % w/w, more preferably in an amount in the range 0.1 - 1.2 % w/w, most preferably in an amount in the range 0. 3 - 1 % w/w. In a particularly preferred embodiment, the at least one acid is present in an amount in the range 0.5 - 0.8 % w/w.

Preferably the at least one acid has a pKa of between 1 and 5, more preferably, between 2 and 4, most preferably about 3.

The at least one acid is present in an amount sufficient to lower the pH of the sanitizing composition to be in the range 2 - 5, more preferably 3 - 4, most preferably, about 3.5.

Preferably, the sanitizing composition further comprises at least one alcohol. Preferably, the at least one alcohol is present in an amount in the range 0.01 - 5 % w/w, more preferably in an amount in the range 0.05 - 2 % w/w, most preferably in an amount in the range 0.15 - 1 % w/w. In a particularly preferred embodiment, the alcohol is present at a concentration in an amount in the range 0.2 - 0.5 % w/w; especially 0.3 - 0.4 % w/w.

Preferably, the at least one alcohol is any C1 to C5, branched or unbranched alcohol. The at least one alcohol may be substituted or unsubstituted. Examples of suitable alcohols include isopropyl alcohol, methanol, ethanol, propan-1-ol, propan-2-ol, butan-1-ol, butan-2-ol, pentan-1-ol, pentan-2-ol, pentan-3-ol, hexan-1-ol, hexan-2-ol, hexan-3-ol, cyclohexanol, cyclopentanol, dimethyl butanol. A preferred at least one alcohol is isopropyl alcohol.

When present, chlorine dioxide is preferably present in an amount in the range 0.001 - 5 % w/w, more preferably, in an amount in the range 0.005 - 1 % w/w, most preferably in an amount in the range 0.01 - 0.5 % w/w. In a particularly preferred embodiment chlorine dioxide is present in an amount in the range of 0.02 - 0.15 % w/w, preferably, in an amount in the range 0.03 - 0.1 % w/w.

The sanitizing composition used in the first aspect preferably comprises water as balance. The water is preferably present in an amount of at least 82 % w/w, preferably at least 90 % w/w, and more preferably at least 92 % w/w. Most preferably it comprises water in amount of at least 95 % w/w. The amount of water present could be up to 100% less the minimum amount of other components, but is preferably up to 99 % w/w (the other components preferably being present in an amount of at least 1 % in total).

The sanitizing compositions used in the first aspect can contain compounds additional to those mentioned above. However preferred compositions of the invention consist essentially of the compounds mentioned above.

A said sanitizing composition used in the first aspect may be used neat, to combat pathogens and biofilms,

It will be appreciated that components of the composition used in the first aspect may be supplied by two different chemical compounds. For example there are two antimicrobial agents (as stated expressly above); there may be two non-ionic surfactants; and so on. When this happens the definitions of the amounts of a particular component given above refer to the total complement of that component, even though that complement is supplied by two (or more) chemical compounds. The same comment applies to definitions given later in relation to further embodiments of the invention.

A concentrate composition may be diluted by water to produce a sanitizing composition used in the first aspect, and capable of destroying pathogens present at a locus and of removing biofilm present at a locus.

Preferably the at least one surfactant is present, in such a concentrate composition, in an amount in the range 2 - 15 % w/w, more preferably in an amount in the range 3 - 10 % w/w, most preferably in an amount in the range 4 - 8 % w/w. In a particularly preferred embodiment, the at least one surfactant is present in an amount in the range 5 - 6 % w/w.

Preferably, the antimicrobial agents are present, in such a concentrate composition, in an amount in the range 0.1 - 12 % w/w, more preferably in an amount in the range 0.5 - 10 % w/w, most preferably in an amount in the range 1 - 7 % w/w. In a particularly preferred embodiment of such a concentrate composition, the antimicrobial agents are present in an amount in the range 2 - 5 % w/w, preferably in an amount in the range 3 - 4 % w/w.

A quaternary ammonium antimicrobial agent is present in such a concentrate composition, and it is preferably present in an amount in at amount in the range 0.01 - 10 % w/w, more preferably in an amount in the range 0.05 - 8 % w/w, more preferably in an amount in the range 0.1 - 6 % w/w, and most preferably in an amount in the range 0.5 - 4% w/w; and especially, in an amount in the range 2 - 3 % w/w.

A biguanide antimicrobial agent is present in such a concentrate composition, and it is preferably present in an amount in an amount in the range 0,01 - 6 % w/w, more preferably in an amount in the range 0.05 - 4 % w/w, more preferably in an amount in the range 0.1 - 3 % w/w, and most preferably in an amount in the range 0.5 - 2 % w/w; and especially, in an amount in the range 1 - 1.5 % w/w.

Preferably the at least one acid is present, in such a concentrate composition, in an amount in the range 3 - 50 % w/w, more preferably in an amount in the range 3 - 45 % w/w, most preferably in an amount in the range 4 - 40 % w/w. In a particularly preferred embodiment, the at least one acid is present in an amount in the range 30 - 40 % w/w, preferably, in an amount in the range 35 - 40 % w/w.

Preferably the at least one acid is present in an amount, in such a concentrate composition, sufficient to lower the pH of the concentrate composition to between 2 and 5, more preferably between 2.5 and 4, most preferably, about 3. Preferably the chlorine dioxide or source thereof (when present) and the acid are introduced to each other, to form the composition of the second embodiment, shortly before use.

Preferably, when the at least one alcohol is present in such a concentrate composition, it is present in an amount in an amount in the range 0.5 - 12 % w/w, more preferably in an amount in the range 1 - 10 % w/w, most preferably in an amount in the range 2 - 8 % w/w. In a particularly preferred embodiment, the alcohol is present at a concentration of about 4 % w/w.

When present in such a concentrate composition, chlorine dioxide is preferably present in an amount, or equivalent amount when it is in stabilised form, in the range 0.05 10 % w/w, more preferably, in an amount in the range 0.1 - 8 % w/w, most preferably in an amount in the range 0.2 - 6 % w/w. In a particularly preferred embodiment chlorine dioxide is present in an amount in the range of 0.5 - 4 % w/w, preferably, in an amount in the range 1 - 3 % w/w.

In a preferred embodiment, such a concentrate composition is produced from a plurality of precursor compositions; preferably, as two precursor compositions.

In such an embodiment, the chlorine dioxide is provided separate from the at least one acid and may be kept in an alkaline solution. Under alkaline conditions, chlorine dioxide (ClO₂) undergoes reduction to chlorite (ClO₂). In this manner chlorine dioxide is stabilized in solution. However, as one would expect, upon acidification of the stabilized chlorine dioxide solution, the chlorite is transformed back to chorine dioxide. Hence chlorine dioxide gas is liberated.

Preferably the concentrate composition described herein yields, on dilution, the sanitizing composition used in the first aspect. Preferably the dilution ratio (concentrate composition:additional water) is in the range 1:1 - 1:100, more preferably 1:4 - 1:50, and still more preferably 1:8 - 1:25. Most preferably the dilution ratio is in the range 1:10 - 1:15; especially preferred is a ratio of 1:13.

Further preferred concentration definitions applicable to the concentrate composition described herein may be derived by taking any of the concentration definitions given above in relation to the sanitizing composition used in the first aspect, and multiplying them by the number 13.

Further preferred concentration definitions applicable to the sanitizing composition used in the first aspect may be derived by taking any of the concentration definitions given above in reflation to said concentrate composition, and dividing them by the number 13.

The "pathogens" killed or disabled in the use of the patent invention are preferably bacteria, and in particular MRSA and/or Legionella. By "disabled" we mean that if the pathogens are not destroyed their essential functioning is disrupted; especially their ability to reproduce and/or grow.

The above invention will now be illustrated by reference to the following examples.

### Example 1

The ability of a sanitizing composition according to the present invention (Composition 1) to neutralise canine parvovirus (CPV) was tested with and without faecal material in order to simulate clean and dirty conditions.

Testing was carried out as follows;
Composition 1 was prepared by the addition of 30 ml of liquid Component A and 30 ml of liquid Component B to 1 litre of distilled water. The solution was stirred together for five minutes then added to 1 litre of water.

### Component A

| | |
|---|---|
| Water | 43% |
| Citric acid | 40% |
| Non-ionic surfactant | 6.5% |
| Fragrance | 0.5% |
| Biguanide(PHMB) | 2% |
| Quaternary ammonium Compound (BAC) | 4% |
| Isopropyl alcohol | 4% |

### Component B

Stabilised chlorine dioxide 2% solution.

Virus was mixed with and without faeces and with and without Composition 1 to give the test material and controls required. Table 1 presents the composition of each control and test solution.

**Table 1 Composition of controls and test solutions**

| | 1 | 2 | 3 | 4 |
|---|---|---|---|---|
| CPV | 1 mL | 1mL | 900µL | 900µL |
| Composition 1 | 1 mL | None | 1 mL | None |
| Faeces | None | None | 0.1g | 0.1g |
| Media | None | 1 mL | None | 1 mL |

For testing under clean conditions, the CPV virus was thawed under cold running water and an equal volume of Composition 1 or cell maintenance media ("Media" herein) added. The composition was mixed thoroughly by rotating. Timing began as soon as Composition 1 was added.

For testing under dirty conditions, 0.1g of normal dog faeces was weighed in to a vessel and 1 mL of CPV virus thawed under cold running water. 900µL of CPV was added to the faeces and the vessel gently shaken until the faecal sample was in suspension. 1mL of Composition 1 or media was added and mixed by rotating the vessel. Timing began as soon as the Composition 1 was added. All reactions were carried out at room temperature.

Samples were collected from each solution at 1, 2, 3, 5, 10, 20, 30 and 60 minute time intervals. At each time interval, a sample was taken and immediately diluted 1 in 10 in media by adding 200µL of the solution to 1800µL of media. The vials were kept on ice. At the end of the hour, the dirty samples were filtered to reduce toxicity to the cell culture and each diluted sample was further diluted by 7 serial 10-fold steps in the following manner: 7 micro centrifuge tubes containing 900µL of media were prepared in advance, 100µL of the 1 in 10 dilution of the test solution or control was taken and pipetted into 900µL of media. The tip was changed, the solution aspirated 8 times and 100µL transferred to the next microcentrifuge tube. This was repeated for each tube in the series.

The control and samples were assessed for the presence of virus by inoculating 50µL of solution into microtitre plates. Each dilution step was added in quadruplicate and then 100µL of CRFK cell suspension (conc 3 x 10⁵) was dispensed into each well and the plates incubated at 37°C for four days.

After 4 days incubation, the plates were fixed, stained and examined for specific fluorescence. The clean solution containing the disinfectant composition was toxic at the 10⁻¹ dilution. A summary of the results is shown in Table 2.

**Table 2 Titres of CPV (Log₁₀ TCID₅₀/50µL) in samples taken from solutions at defined time intervals**

| Time (mins) | Solution 1 | Solution 2 | Solution 3 | Solution 4 |
|---|---|---|---|---|
| 1 | ND | 3.75 | 2.50 | 4.00 |
| 2 | ND | 3.75 | 2.50 | 3.50 |
| 3 | ND | 3.75 | 2.25 | 3.50 |
| 5 | ND | 3.50 | 2.50 | 3.25 |
| 10 | ND | 2.75 | 2.50 | 3.50 |
| 20 | ND | 3.75 | 2.50 | 3.25 |
| 30 | ND | 3.00 | 3.25 | 3.50 |
| 60 | ND | 3.25 | 2.75 | 4.50 |
| ND = No virus detected (≤ 1.5) | | | | |

The preparations contained an appropriate initial concentration of parvovirus. Solutions 2 and 4 record the thermo stability of the virus at room temperature both in culture medium and when diluted with dog faeces (solution 4). Over the 60 minute period of observation there was no significant reduction in viral titre.

Solutions 1 and 3 record the effect of Composition 1. No virus was detected under the clean conditions where virus was mixed with the Composition 1 although the 1st dilution of the test was unreadable due to cell toxicity.

### Conclusion

Composition 1 formulation appears to have a significant effect on canine parvovirus under the test conditions. Composition 1 caused at least a 100 fold reduction on virus titre under the clean test conditions. Under dirty test conditions some virus was detected in the Composition 1 treated solution, but it would appear that there was an approximate 10 fold reduction in viral titre from the control solution.

For Composition 1, significant reduction in titre was observed and it can be concluded that Composition 1 has a good disinfectant effect. The effect seen was rapid (within one minute of addition), and was not increased by further incubation.

### Example 2

### TEST ORGANISMS:

| | |
|---|---|
| Staphylococcus aureus | NCTC 10788 |
| Pseudomonas aeruginosa | NCTC 6749 |
| Escherichia coli | NCTC 10418 |
| Enterococcus hirae | NCTC 12367 |
| Methicillin resistant | NCTC 12493 |
| Staphylococcus aureus | |

### TEST METHOD AND VALIDATION:

EN 1276 Chemical disinfectants and antiseptics - Quantitative suspension test for the evaluation of bactericidal activity of chemical disinfectants and antiseptics used in food, industrial, domestic and institutional areas (Phase 2, step 1).

### REQUIREMENT:

The test product when tested in accordance with the test methodology described under simulated clean and dirty conditions shall demonstrate at least a 5 log 10 reduction.

Product diluent used during the test Sterile hard water 300 mg/kg CaCO₃

### PRODUCT TEST CONCENTRATION:

Undiluted Composition 2 was prepared by mixing 50ml Precursor Composition C and 25ml Precursor Composition D. Precursor Composition C comprised:
600 parts by weight of water;
80 parts by weight SYNPERONIC N (a ethoxylated nonylphenol non-ionic surfactant, from ICI);
200 parts of a citric acid solution that is 50% w/w citric acid in water.
20 parts by weight of PHMB (polyhexamethylbiguanide)
40 parts by weight of BAC (benzyl ammonium chloride)
60 parts by weight of propan-2-ol.

Precursor Composition D comprised a 20,000 ppm solution of chlorine dioxide stabilised by being in alkaline solution (pH 8.5).

Precursor Compositions C (60 ml) and Precursor Composition D (30 ml) were mixed and left for 5 mins, then added to 1 litre of water.

### TEST CONDITIONS:

| | |
|---|---|
| Appearance product dilution | Pale blue solution |
| Contact time | 30 sec, 1, 2 and 5 minutes |
| Test temperature | 20°C |
| Interfering substance: | Bovine albumin |
| | 0.03% clean conditions |
| | 0.03% dirty conditions |
| Inhibition method: | Dilution/neutralization |
| Neutralizer: | Tween 80 40g/l, sodium lauryl sulphate 10g/l, lecithin 4g/l, sodium thiosulphate 5g/l, saponin 30g/litre. |

Tests were performed to establish the suitability of this neutralizer in neutralizing the activity of the disinfectant without being inhibitory to the test organisms. Initial tests were carried out with the standard neutralizer described in EN 1276 but this proved unsatisfactory as a neutralizer. An increase in the Tween 80 and lecithin concentration and the addition of Sodium lauryl sulphate was required.

### SUMMARY OF TEST METHODS:

EN 1276 Chemical disinfectants and antiseptics - Quantitative suspension test for the evaluation of bactericidal activity of chemical disinfectants and antiseptics used in food, industrial, domestic and institutional areas (Phase 2, step 1).

Copies available from BSI, 389 Chiswick High Road, London W4 4AL.

The test method involves mixing 1 ml of the test bacteria with 1 ml of soil (0.3 or 3% albumin and then adding 8 ml of disinfectant. After the required contact time, 1 ml is removed to 9 ml of recovery/neutralizer, which is then plated to detect surviving test bacteria.

### RESULTS

Bactericidal activity of Composition 2 using phase 2 step 1 suspension test EN 1276

**Log₁₀ counts/reductions achieved in 30 secs, 1, 2 and 5 minutes (Tests carried out in duplicate)**

| Log₁₀ reduction | | | | | | | | | |
|---|---|---|---|---|---|---|---|---|---|
| Test organism | Log₁₀ initial count (challenge) | Clean conditions (0.03% albumin) | | | | Dirty conditions (0.03% albumin) | | | |
| | | 30 sec | 1 min | 2 min | 5 min | 30 sec | 1 min | 2 min | 5 min |
| *P. aerug.* | 7.00 | <6.00 | <6.00 | <6.00 | <6.00 | <6.00 | <6.00 | <6.00 | <6.00 |
| *E. coli* | 7.00 | <6.00< | <6.00 | <6.00 | <6.00 | <6.00 | <6.00 | <6.00 | <6.00 |
| *E. Hirae* | 6.15 | <5.15 | <5.15 | <5.15 | <5.15 | <5.15 | <5.15 | <5.15 | <5.15 |
| S. *aureus* | 6.85 | <5.85 | <5.85 | <5.85 | <5.85 | <5.85 | <5.85 | <5.85 | <5.85 |
| *MRSA* | 6.04 | <6.04 | <6.04 | <6.04 | <6.04 | <6.04 | <6.04 | <6.04 | <6.04 |

A contact time of 5 mins is stated in EN 1276. However 30 sec, 1, 2 and 5 mins contact times were employed, since the shorter times were felt to be more relevant for a hard surface disinfectant for use in health care premises. The tests carried out using a shorter contact time were therefore more stringent than as described in EN 1276.

### CONCLUSION

When tested in accordance with EN 1276 (1997), undiluted Composition 2 solution possesses bactericidal activity at 20°C. A >5 Log₁₀ (99.999%) reduction was achieved with all test organisms i.e. *Ps. aeruginosa, Staph. aureus, Esch. coli, Ent. Itirae* and MRSA in 30 secs, 1 min, 2 mins and 5 mins under clean (0.03% albumin) and dirty (0.3% albumin) conditions. To satisfy the requirements for the test, at least a 5 Log₁₀ reduction in specified test organisms is required within 5 mins when the disinfectant is tested at its intended use dilution(s). Composition 2, therefore, satisfies the requirements of the test.

Performance under light (clean) and moderate to heavy (dirty) soiling was assessed. The presence of soil does not appear to affect the performance of the product.

It is believed that a sanitizing composition made in accordance with the present invention effectively and efficiently destroys bacteria and removes biofilm. A sanitizing composition in accordance with the present invention may be used in any situation where the spread of bacteria is unwanted, or the removal of biofilm is required. One such environment is in medical facilities.

## Claims

1. Use of a sanitizing composition both to kill or disable pathogens present at a locus and to destroy or degrade biofilm present at a locus, the composition comprising, in aqueous solution:
at least one surfactant, present in an amount in the range 0.05-5% w/w;
antimicrobial agents;
and at least one acid;
wherein:
said at least one surfactant comprises at least one non-ionic surfactant;
the antimicrobial agents comprise a quaternary ammonium compound present in an amount in the range 0.001-3% w/w, and a biguanide compound present in an amount in the range 0.001-2% w/w;
the pH of the composition is in the range 2-5; and
the at least one acid comprises citric acid.

2. Use as claimed in claim 1, wherein the sanitizing composition contains at least one alcohol.

3. Use as claimed in claim 1 or 2, wherein the non-ionic surfactant comprises an alkoxylated alcohol.

4. Use as claimed in any preceding claim, wherein a concentrate composition is diluted with water to produce the sanitizing composition.

## Patentansprüche

1. Verwenden einer Hygienisierungszusammensetzung zum Abtöten oder zum Unwirksammachen von an einem Ort vorhandenen Pathogenen und zum Zerstören oder Abbauen eines an einem Ort vorhandenen Biofilms, wobei die Zusammensetzung in wässriger Lösung umfasst:
mindestens ein Tensid, das in einer Menge im Bereich von 0,05-5 % Gew./Gew. vorhanden ist;
antimikrobielle Mittel;
und mindestens eine Säure;
wobei:
das mindestens eine Tensid mindestens ein nichtionisches Tensid umfasst;
die antimikrobiellen Mittel eine in einer Menge im Bereich von 0,001-3 % Gew./Gew. vorhandene quaternäre Ammoniumverbindung und eine in einer Menge im Bereich von 0,001-2 % Gew./Gew. vorhandene Biguanidverbindung umfassen;
der pH-Wert der Zusammensetzung im Bereich von 2-5 liegt; und
die mindestens eine Säure Citronensäure umfasst.

2. Verwendung nach Anspruch 1, wobei die Hygienisierungszusammensetzung mindestens einen Alkohol enthält.

3. Verwendung nach Anspruch 1 oder 2, wobei das nichtionische Tensid einen alkoxylierten Alkohol umfasst.

4. Verwendung nach einem der vorhergehenden Ansprüche, wobei eine Konzentratzusammensetzung mit Wasser verdünnt wird, um die Hygienisierungszusammensetzung zu produzieren.

## Revendications

1. Utilisation d'une composition désinfectante pour tuer ou désactiver des pathogènes présents au niveau d'un locus et détruire ou dégrader un biofilm présent au niveau d'un locus, la composition comprenant, en solution aqueuse :
au moins un tensioactif, présent en une quantité dans la plage de 0,05 à 5 % p/p ;
des agents antimicrobiens ;
et au moins un acide ;
dans laquelle :
ledit au moins un tensioactif comprend au moins un tensioactif non ionique ;
les agents antimicrobiens comprennent un composé d'ammonium quaternaire présent en une quantité dans la plage de 0,001 à 3 % p/p, et un composé de biguanide présent en une quantité dans la plage de 0,001 à 2 % p/p ;
le pH de la composition se situe dans la plage de 2 à 5 ; et le au moins un acide comprend de l'acide citrique.

2. Utilisation selon la revendication 1, dans laquelle la composition désinfectante contient au moins un alcool.

3. Utilisation selon la revendication 1 ou 2, dans laquelle le tensioactif non ionique comprend un alcool alcoxylé.

4. Utilisation selon l'une quelconque des revendications précédentes, dans laquelle une composition de concentré est diluée avec de l'eau pour produire la composition désinfectante.
